# EUROPEAN PATENT APPLICATION

(11) **EP 4 843 605 A1**
(43) Date of publication of application: **25.11.2026**
(21) Application number: 25742011.7
(22) Date of filing: 17.01.2025
(51) Int. Cl.: A61M 37/00

(54) **MICRONEEDLE ARRAY**

(30) Priority: 18.01.2024 JP 2024006414
(71) Applicant: Cosmed Pharmaceutical Co., Ltd., Kyoto-shi, Kyoto 601-8438 (JP)
(72) Inventor: QUAN, Ying-shu, Kyoto-shi, Kyoto 601-8438 (JP); LI, Ying-zhe, Kyoto-shi, Kyoto 601-8438 (JP); YAMASHITA, Hirofumi, Kyoto-shi, Kyoto 601-8438 (JP); KAMIYAMA, Fumio, Kyoto-shi, Kyoto 601-8438 (JP)
(74) Representative: Ter Meer und Partner mbB
(86) International application number: PCT/JP2025/001300
(87) International publication number: WO 2025/154786

(57) **Abstract**

The drawback of application type microneedles is that it is difficult to apply a large amount of drug to the microneedles, and thus a drug for effective use is limited, and the drawback of the application type microneedles is eliminated. A drug application type microneedle array including a substrate and a plurality of microneedles, wherein the microneedles are arranged on the substrate so as to form a space by forming a group for every at least three microneedles, and the space provides a drug-applied portion.

## Description

### TECHNICAL FIELD

The present invention relates to a microneedle array having a special structure and arrangement. Further, the present invention relates to a microneedle patch in which an adhesive tape is attached to the microneedle array.

### BACKGROUND ART

Microneedles are currently known in various dosage forms having different shapes and materials, and their properties are also known to be water-soluble and water-insoluble. The applications of the microneedles are also being widely developed in pharmaceuticals and cosmetics and the like.

Regarding the application of the microneedles to a pharmaceutical product, particularly, a vaccine, the microneedles are roughly classified into an encapsulated drug type in the microneedle and an applied drug type in the microneedles. These are called solid microneedles. In consideration of the efficient use of a drug and efficiency in production, the applied drug type is desirable rather than the encapsulated drug type.

A technique is known in which the tip of a microneedle is immersed in a drug solution to attach a drug to the tip of the microneedle (Patent Documents 1 to 4).

### PRIOR ART DOCUMENT

### PATENT DOCUMENTS

Patent Document 1: JP 2008-029710 A
Patent Document 2: JP 2007-521090 A
Patent Document 3: JP 2008-520370 A
Patent Document 4: WO 2008/139648 A

### SUMMARY OF THE INVENTION

### PROBLEM TO BE SOLVED BY THE INVENTION

Since a method for immersing the tip of the microneedle in the drug solution to attach the drug to the tip of the microneedle is simple, it is easy to put the method to practical use. However, it is very difficult to apply the drug to the tip of the microneedle quantitatively and with little variation. In a microneedle made of a hydrophobic material, it is difficult to apply a drug aqueous solution, and when a microneedle made of a hydrophilic material is simply immersed in the drug aqueous solution, the drug aqueous solution easily rises to the bottom surface of a substrate along the needle due to a capillary phenomenon. Therefore, it is extremely difficult to immerse a microneedle array to a certain depth in the drug aqueous solution and apply a quantitatively large amount of drug although many attempts have been made so far.

In order to apply a large amount of drug to the tip portion of the microneedle, it is also known that a water-soluble polymer is added to a drug aqueous solution to be thickened so as to be easily attached to a needle portion (for example, JP 2017-137311 A).

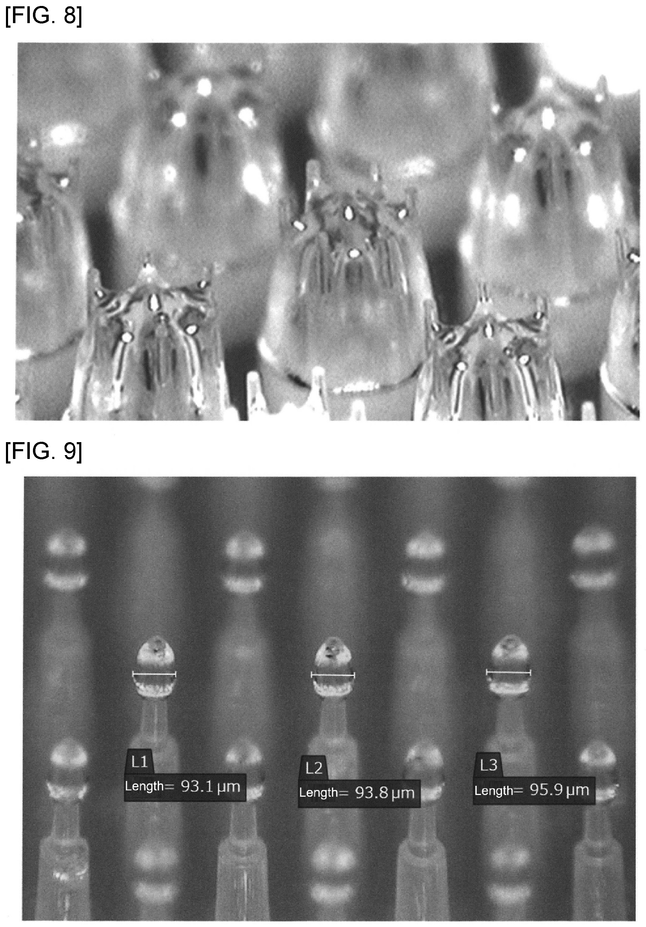

However, it is also pointed out that, by adding the water-soluble polymer, when the drug-applied microneedle array is administered to the skin, it takes time to dissolve a drug-applied portion with a small amount of moisture present in the skin, and the administration time is too long. By increasing the amount of an additive, the drug-applied portion tends to be thick and spherical (see Comparative Example 1 and FIG. 9), a puncture force to the skin remarkably decreases, and the bioavailability of the drug tends to decrease. In a microneedle array in which a drug is simply applied to the tip portion of a microneedle, there are many cases where high drug bioavailability of 80% or more cannot be obtained when the drug amount exceeds 1 mg/cm².

The drawback of application type microneedles is that it is difficult to apply a large amount of drug to the microneedles, and thus the drug for effective use is limited. An object of the present invention is to eliminate the drawback of the application type microneedles.

### MEANS FOR SOLVING THE PROBLEM

In order to solve the above problems, the present inventors have conducted detailed studies on a wide variety of microneedles applied with a drug, and as a result of intensive studies on the geometric shapes of the needles, have found that a microneedle array having a specific shape can achieve a desired object, and have completed the present invention.

The present invention is as follows.
[1] A drug application type microneedle array including a substrate and a plurality of microneedles, wherein the microneedles are arranged on the substrate so as to form a space by forming a group for every at least three microneedles, and the space provides a drug-applied portion.
[2] The microneedle array according to [1], wherein the number of groups of the microneedles is two or more.
[3] The microneedle array according to [1] or [2], wherein the microneedles are made of a thermoplastic polymer.
[4] The microneedle array according to [3], wherein the thermoplastic polymer is nylon, polycarbonate, polylactic acid, a poly(lactic-co-glycolic acid) copolymer, polyglycolic acid, polyethylene terephthalate, a cyclic olefin polymer, and a mixture thereof.
[5] The microneedle array according to any one of [1] to [4], wherein the microneedles have a root diameter of 0.03 to 1 mm, a tip diameter of 0.02 to 0.2 mm, and a length of 0.2 to 3 mm.
[6] The microneedle array according to any one of [1] to [5], wherein a distance between the adjacent microneedles within the group is 0.05 to 1 mm.
[7] The microneedle array according to any one of [1] to [6], wherein the groups of the microneedles are arranged on a base standing on the substrate independently for each group.
[8] The microneedle array according to [7], wherein the base has a cylindrical shape or a truncated cone shape, and the base has a root diameter of 0.1 to 30 mm, a tip diameter of 0.04 to 30 mm, and a height of 0.2 to 10 mm.
[9] The microneedle array according to any one of [1] to [8], wherein the plurality of microneedles are non-uniform in any one of a root diameter, a tip diameter, or a length.
[10] A microneedle patch including the microneedle array according to any one of [1] to [9] and an adhesive tape lined on the substrate of the microneedle array.
[11] A drug application system to skin, including: the microneedle array according to any one of [1] to [9]; and a drug-applied portion provided in the space of the group of the microneedle array.
[12] A drug application system to skin, including: the microneedle patch according to [10]; and a drug-applied portion provided in the space of the group of the microneedle array of the microneedle patch.
[13] The drug application system according to [11] or [12], wherein a plaster retention amount in a space of one group is 10 µg or more.
[14] The drug application system according to any one of [11] to [13], wherein the drug is selected from the group consisting of an antipyretic anti-inflammatory analgesic, a steroidal anti-inflammatory agent, a vasodilator, an arrhythmia agent, a blood pressure lowering agent, a local anesthetic, a hormone agent, an antihistamine, a general anesthetic, a sleep analgesic, an antiepileptic agent, a psychoneurotic agent, a skeletal muscle relaxant, an autonomic nerve agent, an antiparkinsonian agent, a diuretic, a vasoconstrictor, a respiratory promoter, a narcotic, an antigen component of a pathogen, and a cosmetic raw material.
[15] The drug application system according to any one of [11] to [14], wherein a height of the drug-applied portion exceeds a tip of a microneedle.
[16] The drug application system according to any one of [11] to [15], wherein a difference between a tip of the drug-applied portion and a tip of the microneedle is 0.1 m to 2.0 mm.

### EFFECT OF THE INVENTION

The microneedle array and the microneedle patch of the present invention can provide the drug-applied portion as a predetermined space by specifying the arrangement of the microneedles, that is, by grouping the microneedles arranged on the substrate. As a result, the amount of a drug applied was remarkably increased successfully as compared with the conventional microneedle array and microneedle patch. The microneedle array and the microneedle patch of the present invention can be used as a transdermal absorption preparation in which the transdermal delivery amount of the drug is further increased.

### BRIEF DESCRIPTION OF THE DRAWINGS

FIG. 1 is a schematic view showing an example of a microneedle array of the present invention. Three groups of microneedles are arranged on a substrate.
FIG. 2 is a schematic view showing another example of the microneedle array of the present invention. Three groups of microneedles are arranged on separate group bases formed on a substrate.
FIG. 3 is a schematic view showing an example of an application mode when a drug is applied to an application type microneedle array of the present invention.
FIG. 4 is a schematic view showing a microneedle array applied with a drug of the prior art.
FIG. 5 is a micrograph showing a microneedle array of Example 1.
FIG. 6 is an enlarged micrograph of a drug-applied portion when a drug is applied to the microneedle array of Example 1. No drug is applied to a needle tip portion.
FIG. 7 is a micrograph in which a puncture state is reproduced on a parafilm when the application type microneedle array of Example 1 is administered to an upper arm.
FIG. 8 is an enlarged micrograph of a drug-applied portion of an application type microneedle array of Example 2. A drug is applied to a needle tip portion.
FIG. 9 is a micrograph when a drug is applied to a microneedle array of Comparative Example 1.
FIG. 10 is a schematic top view of the microneedle array of the present invention. FIG. 10A shows an aspect in which groups are uniformly distributed, FIG. 10B shows an aspect in which no group is present in a substrate central portion, and FIG. 10C shows an aspect in which groups are coarsely arranged in the substrate central portion and densely arranged in a substrate peripheral portion.
FIG. 11 shows microneedle arrays having microneedles with uniform or non-uniform needle lengths within a group (FIGS. 11A, B), and schematic views after drug application (FIGS. 11C, D). FIGS. 11A and C are examples of microneedles having a uniform needle length, and FIGS. 11B and D are examples of microneedles having a non-uniform needle length.
FIG. 12 is a micrograph showing a microneedle array of Example 3.
FIG. 13 is a micrograph showing an example of a drug application system including the microneedle array of Example 3 and a drug-applied portion having a pointed tip.
FIG. 14 is a micrograph showing an example of a drug application system including the microneedle array of Example 3 and a drug-applied portion having a blunt tip.
FIG. 15 is a schematic view showing the cross-sectional structure of simulated skin in which parafilms are layered.

### MODE FOR CARRYING OUT THE INVENTION

A microneedle array of the present invention is characterized in that, when the tips of microneedles standing on a substrate surface are immersed in a drug solution, a space between tip needles is covered with the surface tension of the drug solution, thereby significantly increasing a drug application amount as compared with a conventional microneedle array.

The microneedle array of the present invention is a drug application type microneedle array. The microneedle array of the present invention includes a substrate and a plurality of microneedles, and the microneedles are arranged on the substrate such that a space provides a drug-applied portion by forming a group for every at least three microneedles to form the space. In the microneedles, the group is formed for every at least three microneedles to form the space. The microneedles are arranged on the substrate such that the space provides the drug-applied portion.

### (Substrate)

The microneedle array of the present invention includes the substrate. The shape of the substrate (shape in plan view) is preferably circular, but is not particularly limited. The diameter of the circular substrate is desirably 1 mm to 30 mm. When the diameter is less than 1 mm, the number of the microneedles standing thereon is small, which makes it difficult to secure a sufficient drug application amount. Considering the planarity of human skin to be punctured, the diameter is desirably 30 mm or less. The thickness of the substrate is 0.05 mm to 3.0 mm, and preferably 0.1 mm to 2.0 mm, from the viewpoint of handling the microneedle array.

### (Patch plate)

The microneedle array of the present invention may or may not include a patch plate on the surface of the substrate on the side opposite to the microneedle side. The patch plate is below the substrate and supports the substrate. The patch plate is provided for molding and storage convenience, but is not an essential portion related to the performance of the microneedle. The patch plate has a larger area than that of the substrate, and has a thickness of 0.5 mm to 2.0 mm.

### (Grouped microneedles)

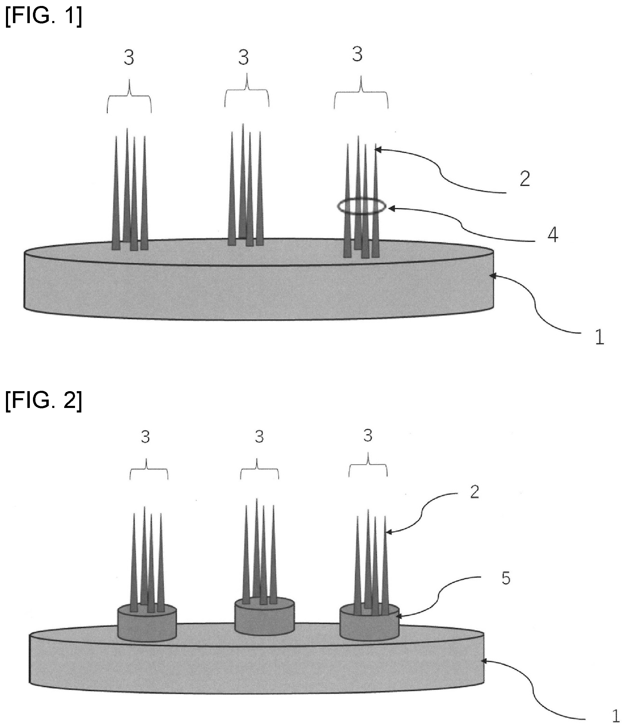

The microneedle array of the present invention includes the plurality of microneedles. The present invention is characterized by the arrangement of the microneedles standing on the substrate of the microneedle array. Although the microneedles form groups and stand on the substrate, each group may stand directly on the substrate (an example thereof is shown in FIG. 1), or each group may form a group base (an example thereof is shown in FIG. 2). Each group has a shape such as a circular shape, a square shape, or a triangular shape, and the microneedles are arranged inside the group. In the case of the circular shape, the diameter is 0.1 mm to 30 mm. When the diameter is less than 0.1 mm, it is difficult to retain three microneedles therein. When the diameter exceeds 30 mm, one group becomes the size of the substrate. When the groups form the base and stand on the substrate, the diameter of the base is equivalent to the above, and the number of the microneedles in each group is 3 to 200, and desirably 3 to 150. The number of the microneedles may be different between the groups. When the number of the microneedles in one group is less than 3, it is difficult to exhibit the characteristic that an applied liquid fills the space between the microneedles, which is an object of the present invention, and the applied liquid is dried to retain the drug between the microneedles. When the number of the microneedles exceeds 200, the needle density increases and the skin puncturability decreases. The structure of the microneedle array is schematically shown in FIG. 1. Although three groups of the microneedles are shown in FIG. 1, there may also be a single group of the microneedles. Usually, the number of the groups is 1 to 1,000, and preferably 2 to 500. When the number exceeds 1,000, the number of the groups is large, and thus the needle density of the microneedles is large, and the skin puncturability is deteriorated.

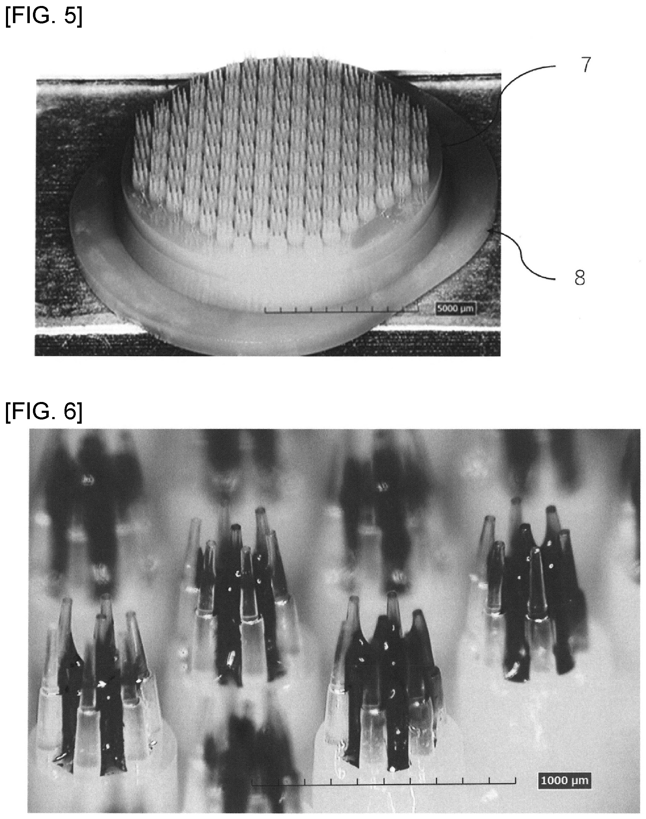

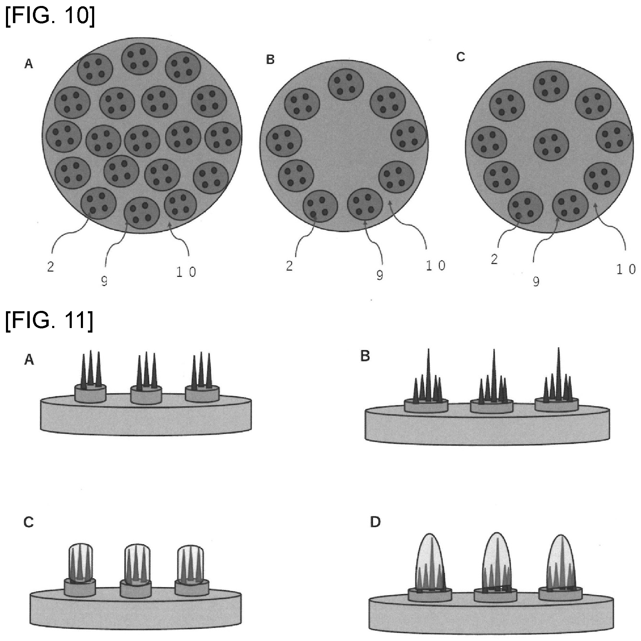

In the present invention, the groups arranged on the substrate of the microneedles need not be uniformly arranged. FIG. 10A shows an example in which the groups are uniformly arranged in the array. In a microneedle array in which a large number of microneedles stand, it is known that puncturability tends to be lower in the central portion of a substrate than in the peripheral portion of the substrate. In the present invention, instead of uniformly arranging the groups on the substrate, the density in the central portion of the substrate may be low (see FIG. 10C) or 0 (see FIG. 10B). The number of the groups in the schematic diagram shown in FIG. 10 is much smaller than the number of the groups shown in the photograph of FIG. 5. Thus, it is also possible to reduce the number of the groups according to the purpose. The number of the microneedles shown in FIG. 10 is 4 microneedles/group, and the number of the microneedles shown in FIG. 5 is 7 microneedles/group. The number of the microneedles is related to the application amount of the drug or the strength of the applied product-containing microneedle, and is desirably changed as necessary.

### (Base)

Although the microneedles form the groups and stand on the substrate, the base may be formed on the substrate, and the microneedles may stand thereon. That is, the microneedle array of the present invention may include the base between the substrate and the microneedles. A structure in which the microneedles stand on the base for each group is schematically shown in FIG. 2. In the case of a substrate on which a base is formed, the microneedles are arranged on the base independently for each group, and are not directly arranged on the substrate. The number of the bases corresponds to the number of the groups of the microneedles, and is 1 to 1,000, and preferably 2 to 500.

The base serves to keep a certain space between the substrate of the microneedle array and the root of the microneedle so that the lower edge of the drug-applied portion does not reach the substrate. The skin has elasticity, and the surface of the skin has irregularities. The present inventors have found that when a microneedle array is administered using an applicator, the microneedle array usually does not entirely enter the root of the needle, but is often in a state of floating by about 200 µm from the root of the needle (see JP 2018-108375 A). Also in the present invention, it is desirable to set the lower edge of the drug-applied portion to a height of 200 µm or more from the substrate of the microneedle array from the viewpoint of drug administration efficiency. In the case of the microneedle array having the base, the lower edge of the drug-applied portion is prevented from reaching the substrate beyond the base, and thus the height of the base can define the lower limit value of the lower edge of the drug-applied portion. Therefore, the height of the base is preferably 0.2 mm to 10 mm.

The presence of the base has an effect of increasing the length of the entire needle and is also advantageous in puncturability. Examples of the shape of the base include a truncated cone shape, a cylindrical shape, and a polygonal frustum shape, and a truncated cone shape and a cylindrical shape are preferable. The upper area of the base is required to stably support the group in which the microneedles stand, and is appropriately determined by the number of the microneedles on the group. It is desirable that the lower area of the base is equal to or larger than the upper area.

When the base is cylindrical or frustoconical, as a suitable size, the root diameter of the base is 0.1 mm to 30 mm, and the tip diameter (the upper surface diameter of the base) is 0.04 mm to 30 mm.

### (Microneedles)

In each group, the arrangement of the microneedles within the group is preferably an arrangement surrounded to retain the applied drug, such as a circular shape, a square shape, or a triangle shape. When puncturing the skin, one group will realize drug delivery to the skin as one drug retainer. The microneedle is preferably a solid microneedle.

The length of the microneedle is 0.2 mm to 3 mm. The length of the microneedle is a length from the needle root to the needle tip portion. The root of the needle is the position of the upper surface of the substrate or the upper surface of the base. When the length is less than 0.2 mm, the volume of a filling portion filling the space between the needles is too small, which deviates from the object of the present invention. In consideration of the moldability of the microneedle and the puncturability into the skin, the length of the microneedle is desirably 3 mm or less.

It is basic that the lengths of the microneedles are all the same, but this is not a requirement. The lengths of the microneedles within the group may be different. The lengths of the plurality of microneedles may be non-uniform. For example, by setting the length of the needle at the central portion of the group to be the longest and decreasing the needle length toward the periphery, the puncturability of the needle can be improved if the shape after drug application in the group is convex at the central portion in the drug application. For example, in FIG. 11, FIG. 11A shows a uniform needle length, and FIG. 11B shows a longer needle in the central portion than in the peripheral portion. When a drug is applied to such a microneedle array, a drug application system shown in FIGS. 11C and D is obtained. In the drug application system of FIG. 11D, the tip of the drug-applied portion is pointed, and thus the drug application system has puncturability and drug bioavailability further improved as compared with the drug application system of FIG. 11C.

The root diameter of the microneedles is 1 mm at the maximum under the condition that the drug solution is filled between the microneedles. The minimum of the root diameter is 0.03 mm, and the mechanical strength of the microneedle is insufficient when the root diameter is less than 0.03 mm. The microneedle may have a step at some midpoint. The root diameters of the plurality of microneedles may be non-uniform.

The tip diameter of the microneedle is 0.02 mm to 0.2 mm. When the tip diameter is less than 0.02 mm, the mechanical strength of the needle becomes weak, which causes a problem in puncturability. When the tip diameter is more than 0.2 mm, the needle is thick, which causes deteriorated skin puncturability. The tip diameters of the plurality of microneedles may be non-uniform.

A distance between the adjacent microneedles within each group (a distance between the adjacent needles) is 0.05 mm to 1 mm. When the distance between the adjacent needles is less than 0.05 mm, the distance does not meet the gist of the present invention in which a large amount of drug is filled between the needles. When the distance between the adjacent needles is 1 mm or less, it is easy to retain the drug solution between the needles by surface tension.

### (Materials of microneedle, substrate, and base)

Examples of the material of the microneedle in the present invention include a thermoplastic polymer that is easily injection-molded or press-molded. The thermoplastic polymer is preferably nylon, polycarbonate, polylactic acid, a poly(lactic-co-glycolic acid) copolymer, polyglycolic acid, polyethylene terephthalate, a cyclic olefin polymer, and a mixture thereof.

The microneedle, the substrate and the base are usually manufactured from the same material, but different materials may be selected.

### (Drug applied to microneedles)

The drug is not particularly limited as long as it is a drug conventionally used as a transdermal absorption preparation and a cosmetic raw material. Examples of the drug include an antipyretic anti-inflammatory analgesic, a steroidal anti-inflammatory agent, a vasodilator, an arrhythmia agent, a blood pressure lowering agent, a local anesthetic, a hormone agent, an antihistamine, a general anesthetic, a sleep analgesic, an antiepileptic agent, a psychoneurotic agent, a skeletal muscle relaxant, an autonomic nerve agent, an antiparkinsonian agent, a diuretic, a vasoconstrictor, a respiratory promoter, a narcotic, and an antigen component of a pathogen (for example, vaccine antigen protein). The content of the drug can be appropriately set according to the characteristics of the component, the purpose of administration, the subject to be administered, and the number of times of administration, and the like.

Many of the drugs are low molecular weight compounds having a molecular weight of 600 or less, but high molecular weight drugs can also be used. Preferable examples of the high molecular weight drugs include physiologically active peptides and derivatives thereof, nucleic acids, oligonucleotides, various antigenic proteins, bacteria, and viral fragments.

Examples of the physiologically active peptides and derivatives thereof include calcitonin, adrenocorticotropic hormone, epidermal growth factor (EGF), parathyroid hormone (PTH), hPTH (1→34), insulin, atrial natriuretic peptide, growth hormone, growth hormone releasing hormone, endothelin, and salts thereof. These drugs can be administered not only for humans but also for animals. Examples of the antigenic proteins include influenza antigen, Japanese encephalitis antigen, diphtheria, tetanus antigen, HBs surface antigen, and HBe antigen.

### (Microneedle patch)

The microneedle array of the present invention can be provided as a microneedle patch by lining an adhesive tape on the substrate of the microneedle array. To apply the microneedle array to the skin, an adhesive tape may be used by being lined around the substrate to fix the array to the skin. The adhesive tape is not essential, but in order to stably fix the patch to the skin, the patch is preferably fixed by the adhesive tape.

The adhesive tape contains an adhesive and a base material, and is obtained by applying the adhesive onto the base material. As the adhesive, rubber-based, silicon-based, acryl-based, and urethane-based adhesives and the like can be used. A hydrophilic adhesive is preferably applied to oral mucosa. Examples of the hydrophilic adhesive include a hydrophilic acrylic adhesive, HiPAS10 (trade name) adhesive (acrylic adhesive containing methyl methacrylate as a main monomer, CosMED Pharmaceutical Co., Ltd.), and HiPAS-PU (trade name) (urethane-based adhesive, CosMED Pharmaceutical Co., Ltd.).

A plasticizer may be added to the adhesive. As the plasticizer, a commonly used plasticizer, for example, isopropyl myristate, isopropyl palmitate, octyl myristate, or octyldodecyl lactate or the like can be used.

### (Manufacture of microneedle array)

The microneedle array can be mass-produced using a template (die). The microneedle array of the injection-moldable thermoplastic polymer may be manufactured by injection-molding a material using a die (for example, the method described in JP 2003-238347 A, [0017], and [0018]). As the injection molding die, stainless steel, heat-resistant steel, or superalloy or the like may be used. A typical die has recesses corresponding to 100 to 1,000 microneedles per square centimeter to form the shape of the microneedle. A fine processing means such as a grinder can be used to form the recesses. Alternatively, a microneedle die is molded by molding using photolithography.

### (Drug application)

The drug is applied to the microneedle by immersing the tip of the microneedle in a drug solution to retain the drug at the tip of the microneedle. It is desirable that the application is performed such that the immersion remains in a needle portion and does not reach a substrate portion. The drug solution is typically an aqueous solution, but may contain a solvent other than water to dissolve the drug. The drug may be completely dissolved or dispersed in a solvent.

In the drug solution, a coexisting substance may be dissolved, and the drug may be retained in the microneedle together with the coexisting substance during drying after application. The coexisting substance is required to be a substance that does not impair the stability of the drug, and for example, water-soluble polymer substances such as hyaluronic acid, collagen, dextrin, dextran, sodium chondroitin sulfate, hydroxypropyl cellulose, ethyl cellulose, a carboxymethyl cellulose sodium salt, and alginic acid, low molecular weight saccharides such as glucose, sucrose, maltose, and trehalose, or mixtures thereof are suitable. The viscosity of the drug solution (applying solution) is preferably 50 mPa·S to 2,000 mPa·S. When the viscosity is too low, the application amount to the microneedle tends to be small, but when the water-soluble polymer substance is not added, the viscosity is adjusted so as to be easily applied with a high content of low molecular weight saccharide solution. When the viscosity is too high, a decrease in fluidity due to the high viscosity makes the application itself difficult.

The drug may be applied by other methods other than the immersion of the tip of the microneedle in the drug solution. For example, a drug solution having high viscosity may be dropped from the upper portion to a microneedle array arranged upward for each group, and the liquid may be rapidly evaporated by ventilation heating or the like to retain the drug and the coexisting substance between the needles. The drug solution may be formed into droplets of an appropriate size, and the droplets may be sprayed and dried for each group by a jet printer to form an applied product composed of a drug and a coexisting substance.

### (Drug application system)

When the tip portions of the microneedles of the microneedle array of the present invention are immersed in the drug solution, the drug solution is retained in a space surrounded by the plurality of microneedles forming the group, and when the drug solution is dried, a drug-applied portion is formed in the space, thus resulting in a drug-applied microneedle array different from the conventional microneedles. The drug application system of the present invention includes the microneedle array or the microneedle patch of the present invention and the drug-applied portion.

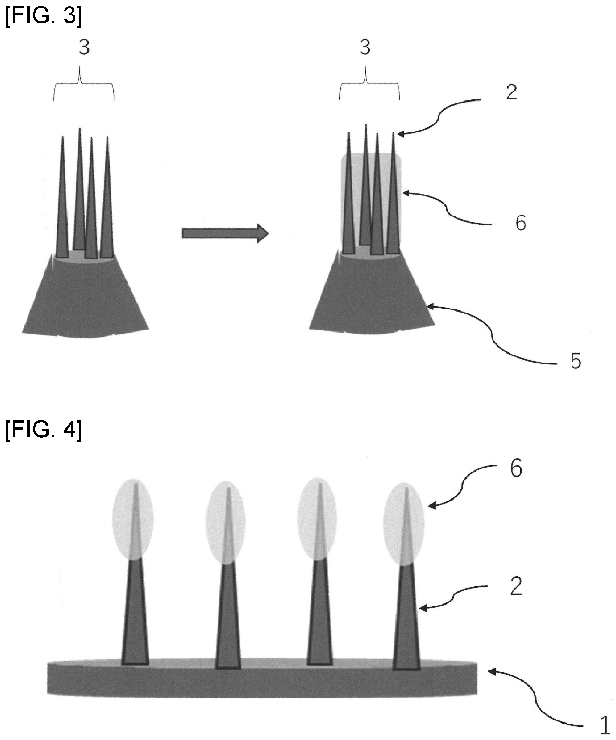

FIG. 3 schematically shows a microneedle array to which a drug is applied. The drug is embedded between the microneedles forming the group, and it is also conceivable that one group functions as one "needle" when puncturing the skin. The microneedle array and the microneedle patch of the present invention having three or more microneedles within one group can largely exert a skin puncture force more than that when the drug is applied to the tip portion of the conventional microneedle to form a large sphere. FIG. 4 schematically shows a conventional microneedle array applied with a drug. The conventional microneedle array has a large spherical application portion, and it is calculated that about 1 µg/needle is a maximum plaster application amount. Here, the plaster is the total applied solid, and is the sum of a drug, a base agent, and a plasticizer and the like. When the content of the drug is 50%, the maximum amount of the drug applied is 0.5 µg/needle.

In the microneedle array of the present invention, the plaster retention amount in the space of one group can be increased to 10 µg or more. The microneedle array and the microneedle patch of the present invention to which the drug is applied are useful as the drug application system to the skin.

In the case of retaining a large amount of drug, it is also possible that the applied shape fills the microneedles surrounded within the maximum group, and in this case, it is also conceivable that the applied shape functions as one "needle" when puncturing the skin. For example, in the microneedle array shown in FIG. 5, all the formed groups can form the drug-applied portion, and the state thereof can be confirmed by the micrograph of FIG. 6.

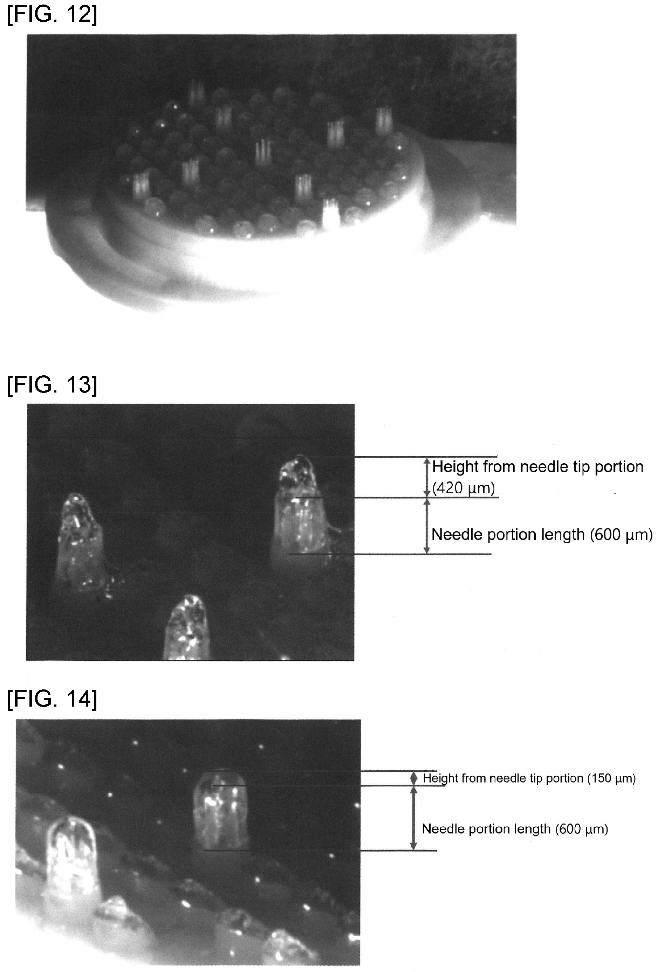

The drug-applied portion for each group shown in FIG. 6 is in a space sandwiched between the microneedles of the group, and the height thereof is equal to or lower than the tip of the microneedle. It is a feature of drug-applied microneedles obtained by normal drug application. However, the height of the drug-applied portion does not need to be equal to or less than the tip of the needle. The height of the drug-applied portion can also be higher than the tip of the needle. By adjusting the concentration of the drug solution to increase the viscosity, a drug-applied portion longer than the needle length can be formed. By forming the tip of the application portion in a pointed shape, the puncturability itself of the microneedle can be improved. As another advantage, making the shape of the application portion higher and sharper than the needle tip inevitably increases the volume of the drug-applied portion, that is, enables an increase in the amount of the drug applied. FIG. 13 shows an example of a drug-applied microneedle in which the drug-applied portion exceeds the tip of the microneedle. Details thereof will be described in Example 3. A difference between the tip of the drug-applied portion and the tip of the microneedle (a distance that the drug-applied portion exceeds the tip of the microneedle) is desirably 0.1 mm to 2 mm.

### EXAMPLES

Hereinafter, the present invention will be described in more detail by the following Examples. These Examples are merely examples for specifically describing the present invention, and the scope of the present invention is not limited to these Examples.

### Example 1

### Microneedle array before drug application

The features of a microneedle array used in Example 1 will be described with reference to FIG. 5.

In the microneedle array shown in FIG. 5, there is a circular substrate 1 having a diameter of 10 mm and a thickness of 1.5 mm on an elliptical patch plate (substrate 2: major axis 1.8 cm, minor axis 1.4 cm, thickness 1 mm), and 109 bases stand thereon. Since the number of microneedles standing on each base was 7, the present microneedle array had 109 bases and 763 microneedles. One of the seven microneedles is arranged at the center of the base, and the remaining six microneedles are arranged around the base. The material of the present microneedle array was polyglycolic acid, and the microneedle array was manufactured by injection molding. One base was a truncated cone having a tip diameter of 0.55 mm, a root diameter of 0.6 mm, and a height of 0.3 mm. A distance between the bases was set such that a distance between the centers of the bases was 0.8 mm. One microneedle had a tip diameter of 0.03 mm, a root diameter of 0.11 mm, and a length of 0.6 mm, and had a step at some midpoint. One microneedle was arranged at the center of the base and six microneedles were arranged around the base so as to surround the one microneedle, and the needles were arranged at equal intervals with a distance between the centers of the needles of 0.2 mm.

### Microneedle array having drug applying portion

The microneedle array manufactured in Example 1 was evaluated as follows.

A trace amount of a red dye (new coccine) was added to an aqueous solution containing 20 mass% of hyaluronic acid to prepare a test solution. The test solution was filled in a wide-mouth container, immersed from the needle tip portion of the microneedle array to a depth of 0.3 mm, pulled up, and dried with hot air. The immersion operation was repeated twice. The micrograph of the tip needle after drying is shown in FIG. 6. The inner space of the six microneedles arranged around the base is filled with the applied product. The weight of the applied product after drying was 5.0 mg as determined by subtracting the weight of the blank microneedle array from the weight of the applied microneedle array. This example is a model experiment, and the weight of the applied product is the plaster weight. When 50% of the drug is contained, the drug content is calculated to be 2.5 mg.

A microneedle array having a drug-applied portion was administered to the human upper arm of a volunteer by an applicator, and taken out after 24 hours. When the microneedle array was dried and the weight was measured, it was found that 90% or more of the applied product (plaster) transferred to the skin.

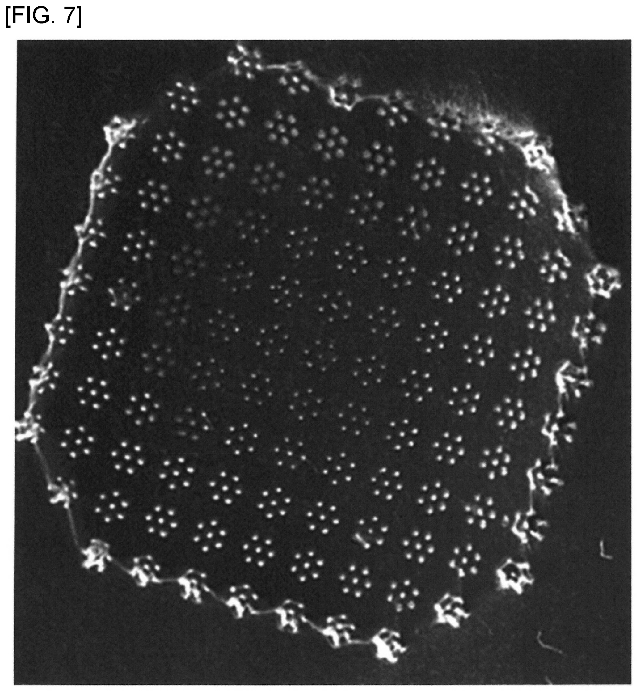

FIG. 7 shows a micrograph of a parafilm when a parafilm (thickness: 140 µm) was placed on the upper arm before the microneedle array of Example 1 was administered to the human upper arm of the volunteer by the applicator, and a microneedle array was separately administered thereon. The insertion traces of all the microneedles were confirmed in the parafilm.

### Example 2

A microneedle array similar to that used in Example 1 was immersed in a test solution similar to that in Example 1 four times in total to manufacture a microneedle array having a drug-applied portion. The weight of the applied product (plaster) after drying was 12.0 mg. The micrograph of the tip needle after drying is shown in FIG. 8.

### Comparative Example 1

An operation of immersing a microneedle array which was made of polyglycolic acid and in which 745 microneedles of the same type as that of the needles of the microneedle array manufactured in Example 1 stood on a substrate having a diameter of 10 mm in a test solution under the same conditions as in Example 1 and drying the microneedle array was repeated twice. The weight of the applied product after drying was measured and found to be 0.45 mg. The micrograph of the microneedle after application is shown in FIG. 9.

### Examples 3 and 4

A part of a microneedle array similar to that used in Example 1 was removed by laser cutting to prepare a microarray including 63 microneedles in 9 groups as shown in FIG. 12. Using this array, the needle surface was directed downward, and a 20 mass% aqueous solution of Metolose (registered trademark, manufactured by Shin-Etsu Chemical Co., Ltd.) was drawn up at a drawing speed of 1 mm/min as an application liquid and dried. This operation was repeated 3 times to prepare a microneedle array (without pointed tip) (Example 4). Thereafter, in fourth application, when a needle tip portion was raised from the application liquid surface by 1 mm, the needle tip portion was stopped, and dried by blowing for 30 seconds to prepare a microneedle array (pointed tip) (Example 3).

Both the micrographs after drying are shown in FIG. 13 (Example 3) and FIG. 14 (Example 4). The physical properties are summarized in Table 1.

**[Table 1]**

| | Height of tip of application portion from needle tip (mm) | Weight of applied product (µg/group) | Parafilm puncturability (µm) |
|---|---|---|---|
| Microneedle array (pointed tip) Example 3 | 0. 43 | 70 | 650 |
| Microneedle array (without pointed tip) Example 4 | 0. 15 | 40 | 390 |

### (Evaluation of parafilm puncturability)

The puncturabilities of the microneedle array (pointed tip) of Example 3 and the microneedle array (without pointed tip) of Example 4 were evaluated.

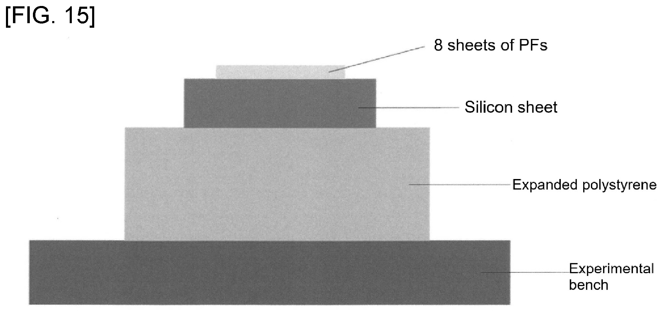

In a puncturability test, 8 sheets of parafilms (PF) were stacked to form simulated skin, and as shown in FIG. 15, an expanded polystyrene sheet, a silicon sheet, and the 8 sheets of PFs (thickness: about 130 µm/sheet) were stacked in this order from the bottom. The silicon sheet and the expanded polystyrene were used to impart a cushioning property similar to that of skin. A microneedle array was applied to the surface of the first sheet (uppermost portion) of the PFs with an in-house applicator, and the number of the PFs through which the microneedles penetrated was evaluated. After the application of the microneedle array, the microneedle array was removed from the PFs. Each PF was observed with a microscope, and an area where the needle penetrated was measured.

As a result, the PF through which all the needles penetrated was up to the fifth sheet (650 µm) from the top in the microneedle array (pointed tip) of Example 3, and up to the third sheet (390 µm) in the microneedle array (without pointed tip) of Example 4.

### Discussion

The microneedle array of Example 1 and the microneedle array of Comparative Example 1 had almost the same number of microneedles, but the drug application amount was 5.0 mg, which was different from 0.45 mg by about 10 times. In the drug-applied portion formed in the microneedle array of Example 1, a large amount of the applied product is efficiently transdermally absorbed, indicating the superiority of the microneedle array of the present invention. It is considered that three or more microneedles are included within one group, and the skin puncture force can be greatly exerted. In Comparative Example 1, a drug-applied portion having a diameter exceeding 90 µm is provided at the tip portion of one microneedle (FIG. 9), and thus it is considered that the transdermal absorption efficiency is low.

The microneedles having a drug applied product having a uniform needle length but a height exceeding the tip of the needle were confirmed to have superiority in puncturability.

### DESCRIPTION OF REFERENCE SYMBOLS

- 1: Substrate
- 2: Microneedle
- 3: Group of microneedles
- 4: Group diameter
- 5: Base
- 6: Drug application portion
- 7: Substrate 1
- 8: Substrate 2 (patch plate)
- 9: Group
- 10: Upper surface of microneedle array

## Claims

1. A drug application type microneedle array including a substrate and a plurality of microneedles, wherein the microneedles are arranged on the substrate so as to form a space by forming a group for every at least three microneedles, and the space provides a drug-applied portion.

2. The microneedle array according to claim 1, wherein the number of groups of the microneedles is two or more.

3. The microneedle array according to claim 1 or 2, wherein the microneedles are made of a thermoplastic polymer.

4. The microneedle array according to claim 3, wherein the thermoplastic polymer is nylon, polycarbonate, polylactic acid, a poly(lactic-co-glycolic acid) copolymer, polyglycolic acid, polyethylene terephthalate, a cyclic olefin polymer, and a mixture thereof.

5. The microneedle array according to any one of claims 1 to 4, wherein the microneedles have a root diameter of 0.03 to 1 mm, a tip diameter of 0.02 to 0.2 mm, and a length of 0.2 to 3 mm.

6. The microneedle array according to any one of claims 1 to 5, wherein a distance between the adjacent microneedles within the group is 0.05 to 1 mm.

7. The microneedle array according to any one of claims 1 to 6, wherein the groups of the microneedles are arranged on a base standing on the substrate independently for each group.

8. The microneedle array according to claim 7, wherein the base has a cylindrical shape or a truncated cone shape, and the base has a root diameter of 0.1 to 30 mm, a tip diameter of 0.04 to 30 mm, and a height of 0.2 to 10 mm.

9. The microneedle array according to any one of claims 1 to 8, wherein the plurality of microneedles are non-uniform in any one of a root diameter, a tip diameter, or a length.

10. A microneedle patch comprising: the microneedle array according to any one of claims 1 to 9; and an adhesive tape lined on the substrate of the microneedle array.

11. A drug application system to skin, comprising: the microneedle array according to any one of claims 1 to 9; and a drug-applied portion provided in the space of the group of the microneedle array.

12. A drug application system to skin, comprising: the microneedle patch according to claim 10; and a drug-applied portion provided in the space of the group of the microneedle array of the microneedle patch.

13. The drug application system according to claim 11 or 12, wherein a plaster retention amount in a space of one group is 10 µg or more.

14. The drug application system according to any one of claims 11 to 13, wherein the drug is selected from the group consisting of an antipyretic anti-inflammatory analgesic, a steroidal anti-inflammatory agent, a vasodilator, an arrhythmia agent, a blood pressure lowering agent, a local anesthetic, a hormone agent, an antihistamine, a general anesthetic, a sleep analgesic, an antiepileptic agent, a psychoneurotic agent, a skeletal muscle relaxant, an autonomic nerve agent, an antiparkinsonian agent, a diuretic, a vasoconstrictor, a respiratory promoter, a narcotic, an antigen component of a pathogen, and a cosmetic raw material.

15. The drug application system according to any one of claims 11 to 14, wherein a height of the drug-applied portion exceeds a tip of a microneedle.

16. The drug application system according to any one of claims 11 to 15, wherein a difference between a tip of the drug-applied portion and a tip of the microneedle is 0.1 m to 2.0 mm.
